Europäisches Patentamt

European Patent Office

Office européen des brevets

(19)

(11) Publication number: **0 001 151**
**B1**

(12) **EUROPEAN PATENT SPECIFICATION**

(45) Date of publication of patent specification: **06.04.83**

(21) Application number: **78200179.6**

(22) Date of filing: **07.09.78**

(51) Int. Cl.³: **A 61 F 7/00, A 61 F 7/08, H 05 B 3/34, A 45 D 2/46**

(54) **Heat treating articles.**

(30) Priority: **09.09.77 US 831839**

(43) Date of publication of application:
**21.03.79 Bulletin 79/6**

(45) Publication of the grant of the patent:
**06.04.83 Bulletin 83/14**

(84) Designated Contracting States:
**BE CH DE FR GB LU NL SE**

(56) References cited:
**DE - C - 580 453**
**GB - A - 427 795**
**US - A - 3 202 801**
**US - A - 3 281 578**

(73) Proprietor: **Bristol-Myers Company**
**Patent Department 345 Park Avenue**
**New York N.Y. 10022 (US)**

(72) Inventor: **Walter, Henry J.**
**41 Longmeadows Road**
**Wilton Connecticut 06897 (US)**
Inventor: **Kunz, Raymond W.**
**20 Maplewood Drive**
**Monroe Connecticut 06468 (US)**

(74) Representative: **Rosenthal, Saul, Dr. et al,**
**c/o Vereenigde Octrooibureaux Nieuwe Parklaan 107**
**NL-2587 BP The Hague (NL)**

Courier Press, Leamington Spa, England

Heat treating articles

This invention relates to a heat treatment device comprising:
a first flexible cover;
a plurality of modules each filled with heat-retaining material;
a heating means; and
a second flexible cover joined with the first flexible cover, said modules and said heating means being confined between said first and second flexible covers respectively.

A heat treatment device of the above defined kind is known from US—A—3,202,801. This prior art relates to an electric heating pad wherein a resistance wire for heating purposes is mounted in a pad body and is surrounded by a closed envelope. The envelope defines flexible chambers or compartments which contain heat retaining material, such as sand or a mineral salt. More particularly this specification discloses a rather simple arrangement of six parallel and relatively large (in proportion to the entire device) chambers.

It is a general object of the subject invention to provide a cap for the heat treatment of hair or scalp of a human's head. In this field of art it is a special problem to provide a heat treatment device of a light weight structure and a high heating efficiency, while providing a comfortable fitting to a user's head.

The above object of the invention is achieved with a heat treatment device which according to the invention is characterized in that said first and second flexible covers have a peripheral shape such as necessary for defining a cap adapted to the shape of a human's head; with the enclosure formed by said first and second flexible covers there is provided a layer of said modules, said modules are disposed in a plurality of waferlike grids throughout the cap surface area, each of said modules being filled with wax; said heating means is ribbon shaped and provides a sealing surface for each of said modules; and an insulating layer is provided between one of said flexible covers and said layer of modules.

The heat treatment device of the subject invention provides a light weight and highly sufficient device which is appropriately adapted to the shape of a human's head.

From US—A—3,281,578 it is known per se to employ a sheet of heat insulating material in an electric mat. However, it is an object of this prior art to provide a snow and ice melting mat having a light weight structure which is flexible so that it can be easily folded or rolled into a compact bundle for storage. Further it is known per se for instance from British patent specification 427,795 to employ wax material and heating means in flexible compresses or covers.

However, the above prior art does not provide a suggestion and/or teaching for coming to a heat treatment device which meet the object of the subject invention.

By employing a ribbon shaped heater in a heat treatment device of the subject invention for each one of the wax-filled modules a sealing surface is provided thereby. Therefore such a ribbon shaped heater contemplates a dual function namely heating and sealing a wax-filled module.

A further contribution to achieve the object of the subject invention a heat treatment device is further characterized thereby that within the enclosure formed by said first and second flexible covers a double layer of wax-filled modules is provided; and the ribbon shaped heating means is disposed between the two layers of modules.

With such a heat treatment device of the subject invention heating is improved while the flex area between adjacent module is halved relative to the total wax thickness.

Embodiments of the subject invention will now be described by way of example with reference to the accompanying drawings, in which:
Fig. 1 is a perspective view of a heat treatment cap of the present invention as it would be applied to a person's head;
Fig. 2 is a perspective view of another heat treatment cap of the present invention;
Fig. 3 is a view of the cap of Fig. 2 in its unassembled condition;
Fig. 4 is a cross-sectional view along the lines IV—IV in Fig. 3; and
Fig. 5 is a view similar to Fig. 4 of another embodiment of a heat treatment cap of the subject invention.

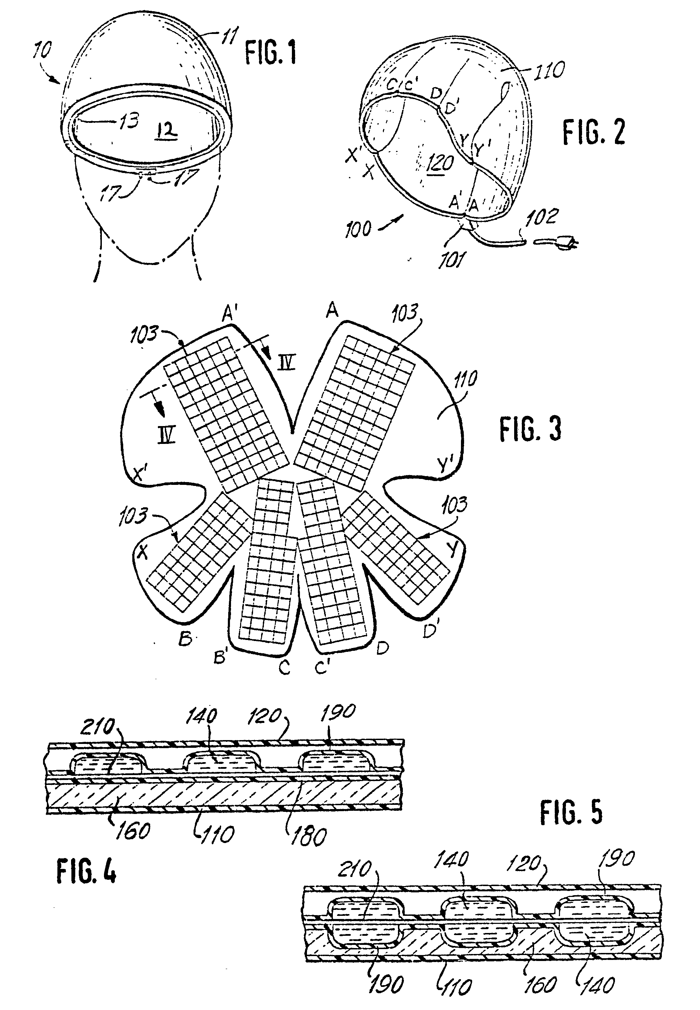

A heat treating device of the invention, namely a hair treatment cap 10, is shown in Fig. 1. The cap has an outer cover 11 and an inner cover 12, which is shown best in and described below with regard to Fig. 2. Covers 11 and 12 are preferably made from vinyl, plastic, or another polymeric material essentially impermeable to moisture.

As shown in Fig. 1, a sanitary cap 13, which is preferably separate from cap 10, may be placed on the user's head prior to the placement thereon of cap 10 to eliminate the need for cleaing cap 10 itself of any heat treatment products which may have been used. Separate cap 13 could be made of an easily washable material to facilitate removal of such products from it. Preferably, cap 13 is made of a single layer of plastic or of a tightly knit material.

Another heat treating cap of this invention, generally indicated at 100, is shown in Fig. 2. This cap utilizes essentially the same components as does the cap of Fig. 1; namely a wax layer, heating elements, and an insulation layer, all enclosed within inner and outer covers.

At the rear portion of cap 100, electrical terminals (not shown) extend outwardly and are received by an electrical connector 101 having a conventional electrical cord 102 attached thereto. The cap has an outer cover 110 and an inner cover 120. Rather than the covers being continuous, as was the case with the covers of the cap of Fig. 1, covers 110 and 120 are discontinuous, as shown in Fig. 3.

Essentially, the periphery of each of the covers is discontinuous because the components of this cap do not extend throughout the cap, as in the cap of Fig. 1, but rather are arranged in numerous grids, each indicated at 103 in Fig. 3. Although the grids are somewhat spaced apart and do not completely cover the cap in its unassembled condition, when the cap is assembled, as shown in Fig. 2, grids 103 become more closely arranged to contact essentially the entire area of the user's head, similar to the cap of Fig. 1.

Referring to Fig. 4, a portion of one grid 103 is shown. Closest to the scalp side of the cap or inner cover 120 is a layer of individual wax modules, each indicated at 140. The modules are formed by barriers 180 and 190, which are sealed at various points. A ribbon shaped heater element 210 is disposed on that side of barrier 180 which faces the wax contained in modules 140. Therefore ribbon shaped heater 210 not only provides a heating function but also functions as a wax barrier. If desired barrier 180 can be omitted. Thus, the heater elements are maintained adjacent to the wax modules. Because of this structure efficient heating of the wax modules is insured. Outwardly of the heating elements is an insulating layer 160 adjacent to outer cover 110.

To use the heat treatment caps of Figs. 1 and 2, the electrical terminals thereof are connected to a power source by the electrical connector and cord, causing the heater elements to be activated. Preferably, after the heater elements have melted the wax, the cap is snugly fit around the user's hair. Once the wax is melted, the electrical connector and cord may be removed. The heat now stored by the wax treats the user's hair as it resolidifies. It has been found that although the inside temperature of the cap may be about 65 to 88°C while the user's hair is being treated, the outside temperature of the cap is generally below 49°C, so that the cap can be easily handled during the treatment. It has also been found that after the termination of power input into the heater elements, the cap is capable of maintaining dry hair at about 54°C for about 45 minutes and moist hair at about 49°C for about 35 minutes. The major reason for these results is the capability of conforming the cap snugly to the particular shape of the user's head to promote most efficient heat transfer thereto from the cap.

It is contemplated that more than one layer of wax modules can be utilized in the caps of this invention and that within a layer on layers of wax modules, waxes having different melting points could be used in different modules to provide for a particular desired multiple temperature operation. Preferably the wax modules are separated by a distance of about twice their thickness to provide maximum flexibility of the cap.

Additionally, a second layer of modules could be used in the arrangement of Fig. 4, similar to that of Fig. 5.

In the arrangement of Fig. 5, wherein heater 210 serves the above two functions, it has been found that with this arrangement, heating is improved while the flex area between the modules is halved relative to the total wax thickness.

## Claims

1. A heat treatment device comprising:
a first flexible cover;
a plurality of modules each filled with heat-retaining material;
a heating means; and
a second flexible cover joined with the first flexible cover, said modules and said heating means being confined between said first and second flexible covers respectively, characterised in that said first and second flexible covers have a peripheral shape such as necessary for defining a cap adapted to the shape of a human's head; within the enclosure formed by said first and second flexible covers there is provided a layer of said modules, said modules are disposed in a plurality of waferlike grids throughout the cap surface area, each of said modules being filled with wax; said heating means is ribbon shaped and provides a sealing surface for each of said modules; and an insulating layer is provided between one of said flexible covers and said layer of modules.

2. A heat treatment device according to claim 1, characterized in that within the enclosure formed by said first and second flexible covers a double layer of wax-filled modules is provided; and the ribbon shaped heating means is disposed between the two layers of modules.

## Patentansprüche

1. Wärmebehandlungsgerät, bestehend aus einer ersten flexiblen Hülle, mehreren, jeweils mit einem wärmespeichernden Material gefüllten Einheiten, einer Heizeinrichtung und einer mit der ersten flexiblen Hülle verbundenen zweiten flexiblen Hülle, wobei die Einheiten und die Heizeinrichtung zwischen den beiden Hüllen eingeschlossen sind, dadurch gekennzeichnet, daß die erste und die zweite flexible Hülle eine für eine der menschlichen Kopfform angepaßte Haube erforderliche Umfangsform aufweisen, daß in dem zwischen der ersten und zweiten flexiblen Hülle gebildeten Zwischenraum eine Schicht der Einheiten vorgesehen ist, daß die Einheiten in mehreren waffelförmigen Gittern überall im Bereich der Kappenfläche angeordnet sind, wobei jede Einheit mit Wachs gefüllt ist, daß die Heizeinrichtung bandförmig ist und eine dichtende Fläche für die Einheiten bildet, und daß zwischen einer der flexiblen Hüllen und der Schicht der Einheiten eine Isolierschicht vorgesehen ist.

2. Wärmebehandlungsgerät nach Anspruch 1, dadurch gekennzeichnet, daß in dem von der ersten und der zweiten flexiblen Hülle gebildeten Zwischenraum eine Doppelschicht aus mit Wachs gefüllten Einheiten vorgesehen ist und daß die bandförmige Heizeinrichtung zwischen den beiden Schichten von Einheiten angeordnet ist.

## Revendications

1. Dispositif de traitement thermique comprenant:
— un premier couvercle flexible;
— une pluralité de modules remplis chacun d'une matière de retenue de chaleur;
— un moyen de chauffage; et
— un second couvercle flexible joint au premier couvercle flexible, lesdits modules et ledit moyen de chauffage étant maintenus respectivement entre lesdits premier et second couvercles flexibles, caractérisé en ce que lesdits premier et second couvercles flexibles ont une forme périphérique telle que celle nécessaire pour définir un chapeau adapté à la forme d'une tête humaine; en ce qu'il est prévu à l'intérieur de l'enveloppe formée par lesdits premier et second couvercles flexibles, une couche desdits modules, ces modules étant disposés dans plusieurs grilles en forme de plaquettes sur la surface du chapeau, chacun desdits modules étant rempli de cire; en ce que ledit moyen de chauffage a une forme de ruban et établit une surface d'étanchéité pour chacun desdits modules; et en ce qu'il est prévu une couche isolante entre un desdits couvercles flexibles et ladite couche de modules.

2. Dispositif de traitement thermique selon la revendication 1, caractérisé en ce qu'il est prévu, à l'intérieur de l'ensemble formé pas lesdits premier et second couvercles flexibles, une double couche de modules remplis de cire et en ce que le moyen de chauffage en forme de ruban est disposé entre les deux couches de modules

FIG. 1

FIG. 2

FIG. 3

FIG. 4

FIG. 5

1